# EUROPEAN PATENT APPLICATION

(11) **EP 4 328 240 A1**
(43) Date of publication of application: **28.02.2024**
(21) Application number: 22382800.5
(22) Date of filing: 25.08.2022
(51) Int. Cl.: C07K 14/47, A23J 3/10, C12N 15/62

(54) **RECOMBINANT PROTEIN PRODUCTION**

(71) Applicant: Real Deal Milk S.L., 08010 Barcelona (ES)
(72) Inventor: The designation of the inventor has not yet been filed
(74) Representative: HGF

(57) **Abstract**

A recombinant fusion protein is provided comprises (a) a casein selected from α-S1 casein, α-S2 casein and β-casein; and (b) a peptide comprising a caseinomacropeptide (CMP), or a fragment thereof. The recombinant fusion protein may be produced by culturing a host cell comprising a nucleic acid encoding the protein. The recombinant fusion protein finds use in the production of a recombinant casein and food compositions comprising casein.

## Description

### Field of the invention

The present invention relates to a recombinant fusion protein, and methods for preparing proteins which utilise a recombinant fusion protein. More particularly, the invention relates to a recombinant fusion protein comprising a casein, and the use of the recombinant fusion protein in preparing caseins.

### Background

Milk (e.g. cows' milk) is an oil-in-water emulsion which comprises dissolved carbohydrates, minerals, lipids and proteins. The proteins present in milk include caseins (α-S1, α-S2, β and κ-caseins) and whey proteins (including β-lactoglobulin, α-lactalbumin, serum albumin and immunoglobulins).

The production of milk proteins, particularly caseins, through the expression of recombinant DNA in a heterologous host system is desirable for multiple ecological, health and animal welfare reasons. For it to be economically viable, the process must occur with high titre (g protein / L culture) and/or high productivity (g protein / number of viable cells in culture).

Well established methods to achieve high level titre and productivity of recombinant proteins in heterologous expression systems, such as expression under a strong promoter or insertion of multiple copies of the recombinant DNA, are not equally effective for all proteins. Specifically, κ-casein responds well to these strategies while α-S1 casein, α-S2 casein and β-casein do not.

Accordingly, there is a need to provide milk proteins such as caseins in a more sustainable and/or economically viable manner.

### Summary of the invention

According to a first aspect of the present invention there is provided a recombinant fusion protein comprising :
(a) a casein selected from α-S1 casein, α-S2 casein and β-casein; and
(b) a peptide comprising a caseinomacropeptide (CMP), or a fragment thereof.

The peptide may increase the hydrophilicity of the recombinant fusion protein relative to the hydrophilicity of the casein alone. In other words, the peptide may decrease the hydrophobicity of the recombinant fusion protein relative to the hydrophobicity of the casein alone.

According to a second aspect of the invention there is provided a nucleic acid molecule comprising a sequence encoding the recombinant fusion protein according to the first aspect of the invention.

According to a third aspect of the invention, there is provided a vector comprising the nucleic acid molecule of the second aspect of the invention.

According to a fourth aspect of the invention, there is provided a host cell comprising the nucleic acid molecule of the second aspect of the invention, or the vector of the third aspect of the invention.

In a fifth aspect, the invention provides a method of producing a recombinant protein, the method comprising culturing the host cell of the fourth aspect of the invention under conditions suitable for expression of the recombinant fusion protein of the first aspect.

In a sixth aspect, the invention provides a method of making a food composition, the method comprising:
(i) providing a recombinant fusion protein according to the first aspect of the invention;
(ii) separating the casein from the peptide; and
(iii) using the separated casein to make a food composition.

In a further aspect, the invention provides a recombinant casein obtained by the method of the fifth aspect.

In yet a further aspect, the invention provides a food composition obtained by the method of the sixth aspect.

In another aspect, the invention provides the use of the recombinant fusion protein of the first aspect in the production of a recombinant casein, and/or in the production of a food composition.

Embodiments of the invention will now be described with reference to the accompanying figures in which:
Figure 1 shows the amino acid sequence of the CMP of bovine κ-casein (uniprot accession number P02668) variant B (referred to herein as SEQ ID NO. 12), excluding the chymosin recognition sequence, with the potential glycosylation sites indicated by arrows;
Figure 2a is a schematic drawing of a recombinant fusion protein in accordance with embodiments of the invention;
Figure 2b is a schematic drawing of a recombinant casein obtained from the recombinant fusion protein of Figure 2a;
Figure 3 is a graph showing the quantity of recombinant α-S1 casein, α-S2 casein, β-casein and κ-casein expressed under a strong promoter in *Pichia pastoris*; and
Figure 4 is a graph showing the quantity of casein (CSN) - caseinomacropeptide (CMP) recombinant fusion proteins expressed using the same promoter, host cell and conditions as in the example shown in Figure 3.

In this specification, the amino acids are designated by the one-letter code, for example, G stands for glycine, P stands for proline, R stands for arginine, etc.

The present invention provides a recombinant fusion protein comprising :
(a) a casein selected from α-S1 casein, α-S2 casein and β-casein; and
(b) a peptide comprising a caseinomacropeptide (CMP), or a fragment thereof.

In some embodiments, the casein is α-S1 casein.

In some embodiments, the casein is α-S2 casein.

In some embodiments, the casein is β-casein.

The α-S1 casein, the α-S2 casein and/or the β-casein may comprise or be constituted by an amino acid sequence which is at least 70%, at least 75%, at least 80%, at least 85%, at least 90%, at least 95%, at least 96%, at least 97%, at least 98%, or at least 99% identical to that of the equivalent wild-type casein protein.

The casein may be derived from any suitable species. For example, the casein (e.g. α-S1 casein, the α-S2 casein and/or the β-casein) may be that of cow, goat, sheep, camel, donkey, horse, human, rat, mouse, guinea pig, pig, water buffalo, rabbit, whale (e.g. fin whale, beluga whale), giraffe or elephant (e.g. African elephant). The accession numbers of casein proteins of representative species are shown in Table 1.

In some embodiments, the α-S1 casein comprises or is constituted by an amino acid sequence which is at least 70%, at least 75%, at least 80%, at least 85%, at least 90%, at least 95%, at least 96%, at least 97%, at least 98%, or at least 99% identical to the sequence of bovine (Bos *taurus*) α-S1 casein (Uniprot accession no. P02662). The bovine α-S1 casein sequence may be variant B.

A representative sequence for bovine α-S1 casein is shown below and is referred to herein as SEQ ID NO. 1. Thus, in some embodiments, the α-S1 casein comprises or is constituted by an amino acid sequence which is at least 70%, at least 75%, at least 80%, at least 85%, at least 90%, at least 95%, at least 96%, at least 97%, at least 98%, or at least 99% identical to SEQ ID NO 1.

RPKHPIKHQGLPQEVLNENLLRFFVAPFPEVFGKEKVNELSKDIGSESTEDQAMEDIKQMEA
ESISSSEEIVPNSVEQKHIQKEDVPSERYLGYLEQLLRLKKYKVPQLEIVPNSAEERLHSMKE
GIHAQQKEPMIGVNQELAYFYPELFRQFYQLDAYPSGAWYYVPLGTQYTDAPSFSDIPNPI
GSENSEKTTMPLW (SEQ ID NO:1)

In some embodiments, the α-S2 casein comprises or is constituted by an amino acid sequence which is at least 70%, at least 75%, at least 80%, at least 85%, at least 90%, at least 95%, at least 96%, at least 97%, at least 98%, or at least 99% identical to the sequence of bovine (Bos *taurus*) α-S2 casein (Uniprot accession no. P02663). The bovine α-S2 casein sequence may be the reference sequence.

A representative sequence for bovine α-S2 casein is shown below and is referred to herein as SEQ ID NO. 2. Thus, in some embodiments, the α-S2 casein comprises or is constituted by an amino acid sequence which is at least 70%, at least 75%, at least 80%, at least 85%, at least 90%, at least 95%, at least 96%, at least 97%, at least 98%, or at least 99% identical to SEQ ID NO 2.

KNTMEHVSSSEESIISQETYKQEKNMAINPSKENLCSTFCKEVVRNANEEEYSIGSSSEESA
EVATEEVKITVDDKHYQKALNEINQFYQKFPQYLQYLYQGPIVLNPWDQVKRNAVPITPTLN
REQLSTSEENSKKTVDMESTEVFTKKTKLTEEEKNRLNFLKKISQRYQKFALPQYLKTVYQH
QKAMKPWIQPKTKVIPYVRYL (SEQ ID NO:2).

In some embodiments, the β-casein comprises or is constituted by an amino acid sequence which is at least 70%, at least 75%, at least 80%, at least 85%, at least 90%, at least 95%, at least 96%, at least 97%, at least 98%, or at least 99% identical to the sequence of bovine (Bos *taurus*) β casein (Uniprot accession no. P02666). In some embodiments, the bovine β casein sequence is variant A2 (the reference). In some embodiments, the bovine β casein sequence is variant A2 (the reference) with the substitution S137R .

A representative sequence for bovine β-casein is shown below and is referred to herein as SEQ ID NO. 3. Thus, in some embodiments, the β casein comprises or is constituted by an amino acid sequence which is at least 70%, at least 75%, at least 80%, at least 85%, at least 90%, at least 95%, at least 96%, at least 97%, at least 98%, or at least 99% identical to SEQ ID NO 3.

RELEELNVPGEIVESLSSSEESITRINKKIEKFQSEEQQQTEDELQDKIHPFAQTQSLVYPFP
GPIPNSLPQNIPPLTQTPVVVPPFLQPEVMGVSKVKEAMAPKHKEMPFPKYPVEPFTERQS
LTLTDVENLHLPLPLLQSWMHQPHQPLPPTVMFPPQSVLSLSQSKVLPVPQKAVPYPQRD
MPIQAFLLYQEPVLGPVRGPFPIIV (SEQ ID NO:3).

The term "identity", with reference to amino acid and nucleic acid sequences, is used herein to describe the level of similarity between two sequences. Sequence similarity or identity may be determined using standard techniques known in the art, including, but not limited to, the local sequence identity algorithm of Smith & Waterman, Adv. Appl. Math. 2, 482 (1981), by the sequence identity alignment algorithm of Needleman & Wunsch, J Mol. Biol. 48,443 (1970), by the search for similarity method of Pearson & Lipman, Proc. Natl. Acad. Sci. USA 85, 2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12, 387-395 (1984), or by inspection. Another suitable algorithm is the BLAST algorithm, described in Altschul et al., J Mol. Biol. 215, 403-410, (1990) and Karlin et al., Proc. Natl. Acad. Sci. USA 90, 5873-5787 (1993).

The recombinant fusion protein of the invention comprises a casein which is fused, directly or indirectly, to a peptide which comprises, or is constituted by, a caseinomacropeptide (CMP), or a fragment thereof.

As used herein, the term "caseinomacropeptide" or "CMP" refers to the C-terminal portion of κ-casein (i.e. the portion which is released upon cleavage of κ-casein by chymosin) but excludes the chymosin recognition sequence. As is known in the art, bovine chymosin cuts κ-casein between an F residue and an M residue (residues 105 and 106 in bovine κ-casein) to release an N-terminal portion (para-kappa casein) and a C-terminal portion (comprising the caseinomacropeptide). The FM motif is contained within a recognition sequence, which in bovine κ-casein is HPHPHLSFMAIPPK (SEQ ID NO:4). Although, post-cleavage, a part of this recognition sequence resides in the C-terminal portion, this part of the recognition sequence is not considered to form a part of the CMP as defined in the context of this specification.

The terms "cleavage site" and "recognition sequence" may be used interchangeably herein to refer to a sequence or chemical motif which is recognised by an enzyme or a chemical capable of cleaving the recombinant fusion protein so as to separate the casein from the CMP.

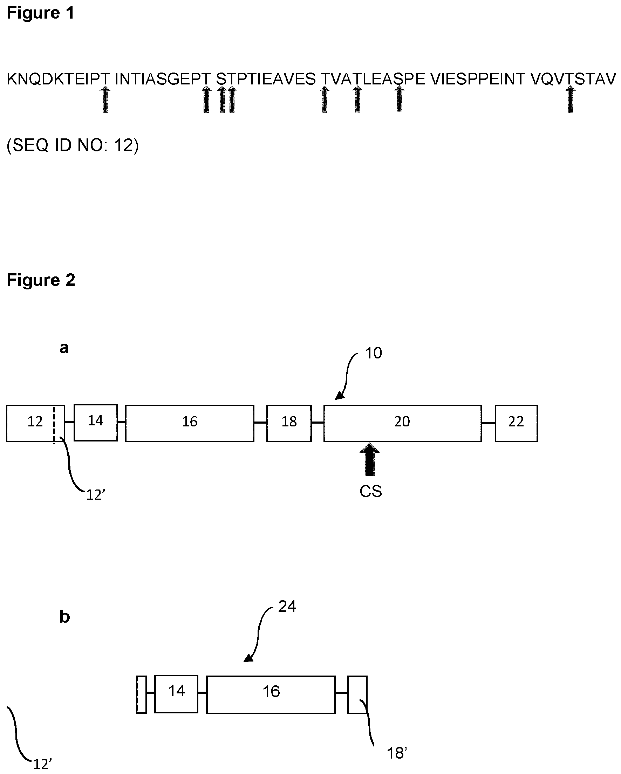

With reference to Figure 1, the CMP of bovine κ-casein has multiple potential glycosylation sites (indicated by arrows) making it highly hydrophilic. Without being bound by theory, it is believed that by fusing the CMP of κ-casein, or a fragment thereof, to other casein proteins (α-S1, α-S2 or β-casein), the resulting fusion protein has improved solubility relative to that of the native caseins, thereby improving the expression of recombinant caseins in a heterologous system.

In some embodiments, the peptide increases the hydrophilicity of the recombinant fusion protein relative to the hydrophilicity of the casein alone. Thus, in some embodiments the peptide is capable of conferring on the recombinant fusion protein an increased hydrophilicity, relative to the hydrophilicity of the (equivalent) casein alone. For example, a recombinant fusion protein comprising α-S1 casein fused to the peptide may have a higher hydrophilicity than the α-S1 casein alone.

It will be understood that increasing the hydrophilicity of the recombinant fusion protein is equivalent to decreasing its hydrophobicity. In some embodiments the peptide decreases the hydrophobicity of the recombinant fusion protein relative to the hydrophobicity of the casein alone. Thus, the peptide may be capable of conferring on the recombinant fusion protein a reduced hydrophobicity, relative to the hydrophobicity of the (equivalent) casein alone. For example, a recombinant fusion protein comprising α-S1 casein fused to the peptide may have a lower hydrophobicity than the α-S1 casein alone.

The hydrophobicity of the recombinant fusion protein and/or the casein may be determined by Hydrophobic Partition, as described in Nakai S. 2003 Measurement of Protein Hydrophobicity; Current Protocols in Food Analytical Chemistry 9(1), Basic Protocol 5.

In some embodiments, the peptide is capable of increasing the yield of the recombinant fusion protein relative to the yield of the (equivalent) casein alone when expressed under identical conditions (e.g. from the same expression cassette, in the same host, under the same environmental conditions).

In some embodiments, the CMP or fragment thereof comprises a sequence of at least 20, at least 25, at least 30, at least 35, at least 40, at least 45 or at least 50 consecutive amino acids.

In some embodiments, the CMP or fragment thereof comprises at least 3, at least 4, at least 5 glycosylation sites. In some embodiments, the CMP or fragment thereof comprises 6 glycosylation sites.

In some embodiments, the CMP comprises an N-terminal or a C-terminal truncation, relative to the full length CMP (as defined herein). In some embodiments, the CMP comprises both an N-terminal and a C-terminal truncation, relative to the full length CMP (as defined herein).

In some embodiments the CMP or fragment thereof comprises:
(i) the amino acid sequence:
   X₃SGX₂PX₁ X₁X₁PTX₃X₂X₃X₃X₂S X₁X₃X₃X₁X₃X₂X₃X₁PX₂ X₃ (SEQ ID NO:5)
   wherein
   each X₁ is independently selected from S and T,
   each X₂ is independently selected from D and E, and
   each X₃ is independently selected from A, I, L and V; or
(ii) an amino acid sequence which is at least 80%, at least 85%, at least 90% or at least 95% identical to SEQ ID NO: 5.

In some embodiments, the CMP or fragment thereof comprises an amino acid sequence which is at least 80%, at least 85%, at least 90% or at least 95% identical to SEQ ID NO: 5, with the proviso that at least 3, at least 4 or at least 5 of the X₁ residues are retained. In some embodiments, all of the X₁ residues are retained.

In some embodiments the CMP or fragment thereof comprises:
(i) an amino acid sequence selected from:
   X₃SGX₂PX₁ X₁X₁PTX₃X₂X₃X₃X₂S X₁X₃X₃X₁X₃X₂X₃X₁PX₂ X₃X₃X₂SPPX₂X₃NT X₃QX₃X₁STX₃X₃ (SEQ ID NO:6); and
   KNQX₂KTX₂X₃PX₁ X₃NTX₃X₃SGX₂PX₁ X₁X₁PTX₃X₂X₃X₃X₂S X₁X₃X₃X₁X₃X₂X₃X₁PX₂ X₃ (SEQ ID NO:7), or
(ii) an amino acid sequence which is at least 70%, at least 75%, at least 80%, at least 85%, at least 90% or at least 95% identical to SEQ ID NO: 6 or SEQ ID NO:7.

In some embodiments, the CMP or fragment thereof comprises an amino acid sequence which is at least 70%, at least 75%, at least 80%, at least 85%, at least 90% or at least 95% identical to SEQ ID NO: 6, with the proviso that at least 3, at least 4, at least 5 or at least 6 of the X₁ residues are retained. In some embodiments, all of the X₁ residues are retained.

In some embodiments, the CMP or fragment thereof comprises an amino acid sequence which is at least 70%, at least 75%, at least 80%, at least 85%, at least 90% or at least 95% identical to SEQ ID NO: 7, with the proviso that at least 3, at least 4, at least 5 or at least 6 of the X₁ residues are retained. In some embodiments, all of the X₁ residues are retained.

In some embodiments the CMP or fragment thereof comprises:
(i) the amino acid sequence 
KNQX2KTX2X3PX1 X3NTX3X3SGX2PX1 X1X1PTX3X2X3X3X2S X1X3X3X1X3X2X3X1PX2
X3X3X2SPPX2X3NT X3QX3X1STX3X3 (SEQ ID NO:8); or
(ii) an amino acid sequence which is at least 70%, at least 75%, at least 80%, at least 85%, at least 90% or at least 95% identical to SEQ ID NO: 8.

In some embodiments, the CMP or fragment thereof comprises an amino acid sequence which is at least 70%, at least 75%, at least 80%, at least 85%, at least 90% or at least 95% identical to SEQ ID NO: 8, with the proviso that at least 3, at least 4, at least 5, at least 6 or at least 7 of the X₁ residues are retained. In some embodiments, all of the X₁ residues are retained.

In some embodiments the CMP or fragment thereof comprises:
(i) the amino acid sequence ASGEPT STPTIEAVES TVATLEASPE V (SEQ ID NO:9); or
(ii) an amino acid sequence which is at least 80%, at least 85%, at least 90% or at least 95% identical to SEQ ID NO: 9.

In some embodiments, the CMP or fragment thereof comprises an amino acid sequence which is at least 80%, at least 85%, at least 90% or at least 95% identical to SEQ ID NO: 9 with the proviso that at least 3, at least 4 or at least 5 glycosylation sites are retained (i.e. present). In some embodiments, 6 glycosylation sites are retained.

In some embodiments the CMP or fragment thereof comprises:
(i) an amino acid sequence selected from:
   ASGEPT STPTIEAVES TVATLEASPE VIESPPEINT VQVTSTAV (SEQ ID NO:10); and
   KNQDKTEIPT INTIASGEPT STPTIEAVES TVATLEASPE V (SEQ ID NO:11), or
(ii) an amino acid sequence which is at least 70%, at least 75%, at least 80%, at least 85%, at least 90% or at least 95% identical to SEQ ID NO: 10 or SEQ ID NO:11.

In some embodiments, the CMP or fragment thereof comprises an amino acid sequence which is at least 70%, at least 75%, at least 80%, at least 85%, at least 90% or at least 95% identical to SEQ ID NO: 10, with the proviso that at least 3, at least 4, at least 5 or at least 6 glycosylation sites are retained (i.e. present). In some embodiments, 7 glycosylation sites are retained.

In some embodiments, the CMP or fragment thereof comprises an amino acid sequence which is at least 70%, at least 75%, at least 80%, at least 85%, at least 90% or at least 95% identical to SEQ ID NO:11, with the proviso that at least 3, at least 4, at least 5 or at least 6 glycosylation sites are retained (i.e. present). In some embodiments, 7 glycosylation sites are retained.

In some embodiments the CMP or fragment thereof comprises or consists of:
(i) the amino acid sequence
   KNQDKTEIPT INTIASGEPT STPTIEAVES TVATLEASPE VIESPPEINT VQVTSTAV (SEQ ID NO:12); or
(ii) an amino acid sequence which is at least 70%, at least 75%, at least 80%, at least 85%, at least 90%, at least 92%, at least 95%, at least 96% or at least 98% identical to SEQ ID NO: 12.

In some embodiments the CMP or fragment thereof comprises (an amino acid sequence which is at least 70%, at least 75%, at least 80%, at least 85%, at least 90%, at least 92%, at least 95%, at least 96% or at least 98% identical to SEQ ID NO: 12, with the proviso that at least 3, at least 4, at least 5, at least 6 or at least 7 glycosylation sites are retained (i.e. present). In some embodiments, 8 glycosylation sites are retained.

In some embodiments, the CMP comprises or is constituted by an amino acid sequence which is at least 70%, at least 75%, at least 80%, at least 85%, at least 90%, at least 95%, at least 96%, at least 97%, at least 98%, or at least 99% identical to the CMP of cow (*Bos taurus*) κ-casein (Uniprot accession no. P02668). The cow κ-casein may be variant B, or it may be the reference variant A or any one of the other variants (B2, E, G, or F).

The amino acid sequence of the CMP from cow κ-casein protein (variant B) is designated herein as SEQ ID NO:12.

The CMP, or fragment thereof, may be derived from any suitable mammal. In some embodiments, the CMP comprises or is constituted by an amino acid sequence which is at least 70%, at least 75%, at least 80%, at least 85%, at least 90%, at least 95%, at least 96%, at least 97%, at least 98%, or at least 99% identical to the wild-type CMP of κ-casein from goat, sheep, camel, donkey, horse, human, rat, mouse, guinea pig, pig, water buffalo, rabbit, whale (e.g. fin whale, beluga whale), giraffe or elephant. The accession numbers of κ-casein for exemplary species are shown in Table 1.

**Table 1**

| | **Accession no. (uniprot)** | | | |
|---|---|---|---|---|
| **Species** | **αs1 casein** | **αs2 casein** | **β casein** | **κ casein** |
| Cow (*Bos taurus*) | P02662 | P02663 | P02666 | P02668 |
| Goat | P18626 | P33049 | P33048 | P02670 |
| Sheep | P04653 | P04654 | P11839 | P02669 |
| Camel | 097943 | 097944 | Q9TVD0 | P79139 |
| Donkey | P86272 | B7VGF9 | P86273 | B1PLB8 |
| Human | P47710 | | P05814 | P07498 |
| Horse | Q8SPR1 | D2KAS0 | Q9GKK3 | P82187 |
| Rat | P02661 | | P02665 | P04468 |
| Mouse | P19228 | | P10598 | P06796 |
| Guinea pig | P04656 | P04655 | | P19442 |
| Pig | P39035 | P39036 | P39037 | P11841 |
| Domestic water buffalo | 062823 | E9NZN2 | Q9TSI0 | P11840 |
| Rabbit | P09115 | P50419 | P09116 | P33618 |
| Fin whale | | A0A6A1Q9C4 | Q27953 | Q27952 |
| Beluga whale | A0A7F8K8A0 | A0A7F8K156 | A0A2Y9M8L5 | A0A2Y9M6S7 |
| Giraffe | | | Q28418 | Q28417 |
| African elephant | | | G3TDS4 | G3SNT0 |

As used herein, the term "fusion" will be understood to mean that the casein and the peptide are joined directly or indirectly via peptide bonds such that they form two portions of the same recombinant protein.

In some embodiments, the recombinant fusion protein comprises a linker between the casein and the peptide.

The linker may comprise an amino acid sequence of from 3 to 50, from 5 to 40, from 8 to 30, from 10 to 20 or from 12 to 15 residues in length.

In some embodiments, the linker comprises a cleavage site. Advantageously, the cleavage site can be used to cleave the recombinant fusion protein so as to separate the casein from the peptide. The cleavage site may be a chemical cleavage site or an enzyme cleavage site.

In some embodiments, the cleavage site is an enzyme cleavage site. The enzyme cleavage site may be a hydrolase cleavage site (such as a protease cleavage site) or a peptide lyase cleavage site (such as an amidine lyase cleavage site).

In some embodiments the cleavage site is a protease cleavage site. The cleavage site may be a chymosin cleavage site, or an enteropeptidase cleavage site.

A suitable enteropeptidase cleavage site may be that of bovine enteropeptidase (Uniprot accession number P98072).

In some embodiments, the cleavage site is a chymosin cleavage site. The chymosin cleavage site may be that of any mammal. For example, the chymosin cleavage site may be bovine, goat, sheep, or camel. In some embodiments, the cleavage site is derived from the same species as the CMP.

In some embodiments the chymosin cleavage site comprises the amino acid sequence HLSFXAIP (SEQ ID NO.13), wherein X is L, M or F. Cleavage occurs between the F and X residues.

Some additional residues are believed to make the interaction with the enzyme more stable. Therefore, in some embodiments the chymosin cleavage site comprises the sequence HPHPHLSFMAIPPK (SEQ ID NO.4).

In some embodiments, the chymosin cleavage site comprises the sequence FLQKQQYGISSKFX wherein X is selected from: W, Y, F, C, R, I, M, H, L, V, K, N, T or A (SEQ ID NO.14). Preferably, X is W, Y or F.

In some embodiments, the chymosin cleavage site comprises the sequence RPRPRPSFIAIPPK (SEQ ID NO.15).

Other suitable cleavage sites which may be incorporated into the recombinant fusion protein will be known to those skilled in the art, such as those described in WO2022/072718.

The casein may be located at or towards the N-terminal end of the recombinant fusion protein, and the peptide may be located at or towards the C-terminal end of the recombinant fusion protein. Alternatively, the casein may be located at or towards the C-terminal end of the recombinant fusion protein, and the peptide may be located at or towards the N-terminal end of the recombinant fusion protein.

In some embodiments, the casein is located at or towards the N-terminal end, and the peptide at or towards the C-terminal end of the recombinant fusion protein.

The recombinant fusion protein may further comprise additional features, such as one or more tags (e.g. a HIS tag), and/or a signal sequence.

It will be appreciated that the components of the recombinant fusion protein (i.e. the casein, the peptide, and any tag(s) and/or signal sequences, if present) may be directly joined to each other via peptide bonds, or they may be joined via one or more linkers.

In some embodiments, the recombinant fusion protein comprises a signal sequence or a fragment thereof. As is known in the art, a signal sequence (also referred to as a "signal peptide", "leader sequence", "signal leader", "targeting signal", "localization signal" or "localization sequence") is a short amino acid sequence that is normally present at the N-terminus of a protein, and which prompts the producing cell to translocate the protein through a membrane. A secretory signal sequence prompts translocation through the membrane of the endoplasmic reticulum (in eukaryotic organisms) and therefore into the secretory pathway. Signal sequences may be cleaved during or after translocation of the protein through the target membrane to generate a free signal peptide and a mature protein. Complex leader sequences can comprise multiple processing (i.e. cleavage) positions. A first (N-terminal) portion, which strictly speaking is the localization sequence, is referred to as pre-sequence (or "pre-signal", "pre-region", "pre-peptide") and is cleaved during translocation or immediately after translocation. A further portion, referred to as pro-sequence (or "pro-signal", "pro-peptide", "pro-region"), is believed to modulate progression through the pathway and is cleaved at later stages of transit through the secretory pathway. Incomplete processing of a complex leader sequence may result in the secreted protein retaining some amino acids from the pro-sequence at its N terminus. Thus, in some embodiments the recombinant fusion protein comprises at its N terminus at least some amino acids from the pro-region of the signal sequence.

The signal sequence may be any suitable signal that is known in the art. The skilled person is capable of selecting a signal sequence which is appropriate for the host cell in which the recombinant fusion protein is intended to be expressed.

In some embodiments, the signal sequence is selected from: S. *cerevisiae* α-mating factor (MF-α) signal sequence or a chimera, fragment or variant (e.g. comprising point mutations) thereof.

In some embodiments, the signal sequence is the *S*. *cerevisiae* α-mating factor (MF-α) signal sequence.

In some embodiments, the signal sequence is a chimeric signal sequence comprising the pre-region of the *S*. *cerevisiae* Ost1 signal sequence and the pro-region of the *S*. *cerevisiae* MF-α signal sequence.

In some embodiments, the signal sequence comprises a sequence which is at least 90%, at least 95%, at least 96%, at least 97%, at least 98% or at least 99% identical to SEQ ID NO. 16.

MRFPSIFTAVLFAASSALAAPVNTTTEDETAQIPAEAVIGYSDLEGDFDVAVLPFSNSTNNGL
LFINTTIASIAAKEEGVSLEKREAEA (SEQ ID NO:16).

In some embodiments, the recombinant fusion protein comprises or has the structure, from N-terminus to C-terminus:
Signal sequence (or fragment thereof) - tag - casein - linker - CMP (or fragment thereof) - tag, wherein the linker comprises a cleavage site (e.g. a chymosin cleavage site).

In some embodiments, the recombinant fusion protein comprises or has the structure, from N-terminus to C-terminus:
Signal sequence (or fragment thereof) - casein - linker - CMP (or fragment thereof), wherein the linker comprises a cleavage site (e.g. a chymosin cleavage site).

In some embodiments, the recombinant fusion protein comprises or has the structure, from N-terminus to C-terminus:
Casein - linker - CMP (or fragment thereof), wherein the linker comprises a cleavage site (e.g. a chymosin cleavage site).

In an embodiment, the recombinant fusion protein comprises or consists of an amino acid sequence which is at least 70%, at least 75%, at least 80%, at least 85%, at least 90%, at least 95%, at least 96%, at least 97%, at least 98% or at least 99% identical to SEQ ID NO: 17 or SEQ ID NO:18. In an embodiment, the recombinant fusion protein comprises or consists of an amino acid sequence according to SEQ ID NO:17 or SEQ ID NO:18. The recombinant fusion protein comprises bovine α-S1 casein fused via a linker to the CMP peptide of bovine k-casein, wherein the linker comprises the bovine chymosin cleavage site. The sequence of SEQ ID NO: 17 comprises the *S*. *cerevisiae* α-mating factor (MF-α) signal sequence at its N-terminus. During secretion of the recombinant fusion protein by the host cell, this signal sequence is cleaved, leaving behind a small fragment thereof on the secreted protein. The secreted protein is represented by SEQ ID NO:18.

Expressed fusion protein comprising α-S1 casein-linker-CMP:

MRFPSIFTAVLFAASSALAAPVNTTTEDETAQIPAEAVIGYSDLEGDFDVAVLPFSNSTNNGL
LFINTTIASIAAKEEGVSLEKREAEAHHHHHHRPKHPIKHQGLPQEVLNENLLRFFVAPFPEV
FGKEKVNELSKDIGSESTEDQAMEDIKQMEAESISSSEEIVPNSVEQKHIQKEDVPSERYLG
YLEQLLRLKKYKVPQLEIVPNSAEERLHSMKEGIHAQQKEPMIGVNQELAYFYPELFRQFYQ
LDAYPSGAWYYVPLGTQYTDAPSFSDIPNPIGSENSEKTTMPLWHPHPHLSFMAIPPKKNQ
DKTEIPTINTIASGEPTSTPTIEAVESTVATLEASPEVIESPPEINTVQVTSTAVGSGSHHHHH
H (SEQ ID NO:17)

Secreted fusion protein comprising α-S1 casein-linker-CMP:

EAEAHHHHHHRPKHPIKHQGLPQEVLNENLLRFFVAPFPEVFGKEKVNELSKDIGSESTED
QAMEDIKQMEAESISSSEEIVPNSVEQKHIQKEDVPSERYLGYLEQLLRLKKYKVPQLEIVPN
SAEERLHSMKEGIHAQQKEPMIGVNQELAYFYPELFRQFYQLDAYPSGAWYYVPLGTQYT
DAPSFSDIPNPIGSENSEKTTMPLWHPHPHLSFMAIPPKKNQDKTEIPTINTIASGEPTSTPTI
EAVESTVATLEASPEVIESPPEINTVQVTSTAVGSGSHHHHHH (SEQ ID NO:18).

In an embodiment, the recombinant fusion protein comprises or consists of an amino acid sequence which is at least 70%, at least 75%, at least 80%, at least 85%, at least 90%, at least 95%, at least 96%, at least 97%, at least 98% or at least 99% identical to SEQ ID NO. 19 or SEQ ID NO. 20. In an embodiment, the recombinant fusion protein comprises or consists of an amino acid sequence according to SEQ ID NO. 19 or SEQ ID NO:20. The recombinant fusion protein comprises bovine α-S2 casein fused via a linker to the CMP peptide of bovine k-casein, wherein the linker comprises the bovine chymosin cleavage site. The sequence of SEQ ID NO:19 comprises the *S*. *cerevisiae* α-mating factor (MF-α) signal sequence at its N-terminus. During secretion of the recombinant fusion protein by the host cell, this signal sequence is cleaved, leaving behind a small fragment thereof on the secreted protein. The secreted protein is represented by SEQ ID NO:20.

Expressed fusion protein comprising α-S2 casein-linker-CMP:

MRFPSIFTAVLFAASSALAAPVNTTTEDETAQIPAEAVIGYSDLEGDFDVAVLPFSNSTNNGL
LFINTTIASIAAKEEGVSLEKREAEAHHHHHHKNTMEHVSSSEESIISQETYKQEKNMAINPS
KENLCSTFCKEVVRNANEEEYSIGSSSEESAEVATEEVKITVDDKHYQKALNEINQFYQKFP
QYLQYLYQGPIVLNPWDQVKRNAVPITPTLNREQLSTSEENSKKTVDMESTEVFTKKTKLTE
EEKNRLNFLKKISQRYQKFALPQYLKTVYQHQKAMKPWIQPKTKVIPYVRYLHPHPHLSFMA
IPPKKNQDKTEIPTINTIASGEPTSTPTIEAVESTVATLEASPEVIESPPEINTVQVTSTAVGSG
SHHHHHH (SEQ ID NO:19).

Secreted fusion protein comprising α-S2 casein-linker-CMP:

EAEAHHHHHHKNTMEHVSSSEESIISQETYKQEKNMAINPSKENLCSTFCKEVVRNANEEE
YSIGSSSEESAEVATEEVKITVDDKHYQKALNEINQFYQKFPQYLQYLYQGPIVLNPWDQVK
RNAVPITPTLNREQLSTSEENSKKTVDMESTEVFTKKTKLTEEEKNRLNFLKKISQRYQKFAL
PQYLKTVYQHQKAMKPWIQPKTKVIPYVRYLHPHPHLSFMAIPPKKNQDKTEIPTINTIASGE
PTSTPTIEAVESTVATLEASPEVIESPPEINTVQVTSTAVGSGSHHHHHH (SEQ ID NO:20).

In an embodiment, the recombinant fusion protein comprises or consists of an amino acid sequence which is at least 70%, at least 75%, at least 80%, at least 85%, at least 90%, at least 95%, at least 96%, at least 97%, at least 98% or at least 99% identical to SEQ ID NO. 21 or SEQ ID NO:22. In an embodiment, the recombinant fusion protein comprises or consists of an amino acid sequence according to SEQ ID NO. 21 or SEQ ID NO:22. The recombinant fusion protein comprises bovine β-casein fused via a linker to the CMP peptide of bovine k-casein, wherein the linker comprises the bovine chymosin cleavage site. The sequence of SEQ ID NO:21 comprises the *S*. *cerevisiae* α-mating factor (MF-α) signal sequence at its N-terminus. During secretion of the recombinant fusion protein by the host cell, this signal sequence is cleaved, leaving behind a small fragment thereof on the secreted protein. The secreted protein is represented by SEQ ID NO:22.

Expressed fusion protein comprising β-casein-linker-CMP:

MRFPSIFTAVLFAASSALAAPVNTTTEDETAQIPAEAVIGYSDLEGDFDVAVLPFSNSTNNGL
LFINTTIASIAAKEEGVSLEKREAEAHHHHHHRELEELNVPGEIVESLSSSEESITRINKKIEKF
QSEEQQQTEDELQDKIHPFAQTQSLVYPFPGPIPNSLPQNIPPLTQTPVVVPPFLQPEVMGV
SKVKEAMAPKHKEMPFPKYPVEPFTERQSLTLTDVENLHLPLPLLQSWMHQPHQPLPPTV
MFPPQSVLSLSQSKVLPVPQKAVPYPQRDMPIQAFLLYQEPVLGPVRGPFPIIVHPHPHLSF
MAIPPKKNQDKTEIPTINTIASGEPTSTPTIEAVESTVATLEASPEVIESPPEINTVQVTSTAVG
SGSHHHHHH (SEQ ID NO:21).

Secreted fusion protein comprising β-casein-linker-CMP:

EAEAHHHHHHRELEELNVPGEIVESLSSSEESITRINKKIEKFQSEEQQQTEDELQDKIHPFA
QTQSLVYPFPGPIPNSLPQNIPPLTQTPVVVPPFLQPEVMGVSKVKEAMAPKHKEMPFPKY
PVEPFTERQSLTLTDVENLHLPLPLLQSWMHQPHQPLPPTVMFPPQSVLSLSQSKVLPVPQ
KAVPYPQRDMPIQAFLLYQEPVLGPVRGPFPIIVHPHPHLSFMAIPPKKNQDKTEIPTINTIAS
GEPTSTPTIEAVESTVATLEASPEVIESPPEINTVQVTSTAVGSGSHHHHHH (SEQ ID
NO:22).

The invention further provides a nucleic acid molecule comprising a sequence encoding a recombinant fusion protein as described herein.

The nucleic acid molecule may be DNA or RNA. In some embodiments the nucleic acid molecule is DNA.

In some embodiments the nucleic acid molecule comprises a sequence selected from SEQ ID NO:23, SEQ ID NO:24 or SEQ ID NO:25:
DNA sequence encoding fusion protein comprising α-S1 casein-linker-CMP: 
ATGAGATTTCCATCTATTTTCACTGCTGTTTTGTTTGCTGCTTCTTCTGCTTTGGCTGCTC
CAGTTAACACTACTACTGAAGATGAGACTGCTCAAATTCCTGCTGAAGCTGTTATTGGTT
ACTCTGATTTGGAGGGAGATTTTGATGTTGCTGTTTTGCCTTTCTCTAACTCTACTAACA
ACGGTTTGTTGTTCATTAACACTACTATCGCTTCTATCGCTGCTAAGGAAGAGGGTGTTT
CTTTGGAAAAGAGAGAAGCTGAGGCTCACCATCACCACCATCACAGACCAAAGCATCC
TATTAAACACCAAGGTTTGCCACAAGAAGTTTTGAACGAGAATTTGTTGAGATTTTTCGT
 
TGCTCCATTTCCTGAAGTTTTCGGTAAAGAAAAAGTTAACGAGTTGTCTAAGGATATTGG
TTCTGAATCTACTGAGGATCAAGCTATGGAAGATATCAAGCAAATGGAAGCTGAGTCTA
TCTCTTCTTCTGAAGAGATTGTTCCAAACTCTGTTGAACAAAAGCATATCCAAAAGGAAG
ATGTTCCTTCTGAGAGATACTTGGGTTATTTGGAACAATTGTTGAGATTGAAGAAATACA
AGGTTCCACAATTGGAGATTGTTCCTAACTCTGCTGAAGAGAGATTGCATTCTATGAAG
GAAGGTATTCACGCTCAACAAAAAGAGCCAATGATTGGTGTTAATCAAGAATTGGCTTA
CTTCTACCCTGAGTTGTTTAGACAATTCTACCAATTGGATGCTTATCCATCTGGTGCTTG
GTACTATGTTCCTTTGGGTACTCAATACACTGATGCTCCATCTTTCTCTGATATTCCAAA
CCCTATTGGTTCTGAAAATTCTGAGAAAACTACTATGCCTTTGTGGCATCCACACCCTCA
TTTGTCTTTCATGGCTATTCCACCTAAGAAAAACCAAGATAAGACTGAAATCCCAACTAT
TAATACTATTGCTTCTGGAGAGCCAACTTCTACTCCTACTATTGAAGCTGTTGAGTCTAC
TGTTGCTACTTTGGAAGCTTCTCCTGAAGTTATTGAGTCTCCACCTGAGATTAATACTGT
TCAAGTTACTTCTACTGCTGTTGGTTCTGGATCCCATCACCATCACCATCACTAA (SEQ
ID NO:23).
DNA sequence encoding fusion protein comprising α-S2 casein-linker-CMP: 
ATGAGATTTCCATCTATTTTCACTGCTGTTTTGTTTGCTGCTTCTTCTGCTTTGGCTGCTC
CAGTTAACACTACTACTGAAGATGAGACTGCTCAAATTCCTGCTGAAGCTGTTATTGGTT
ACTCTGATTTGGAGGGAGATTTTGATGTTGCTGTTTTGCCTTTCTCTAACTCTACTAACA
ACGGTTTGTTGTTCATTAACACTACTATCGCTTCTATCGCTGCTAAGGAAGAGGGTGTTT
CTTTGGAAAAGAGAGAAGCTGAGGCTCACCATCACCACCATCACAAGAACACTATGGAA
CATGTTTCTTCTTCTGAAGAGTCTATCATCTCTCAAGAAACTTACAAGCAAGAGAAAAAC
ATGGCTATTAATCCATCTAAGGAAAATTTGTGTTCTACTTTCTGTAAGGAGGTTGTTAGA
AACGCTAATGAAGAGGAATACTCTATTGGTTCTTCTTCTGAGGAATCTGCTGAAGTTGCT
ACTGAGGAAGTTAAGATCACTGTTGATGATAAGCACTACCAAAAGGCTTTGAACGAGAT
TAATCAATTCTACCAAAAGTTCCCTCAATATTTGCAATACTTGTATCAAGGTCCAATTGTT
TTGAACCCTTGGGATCAAGTTAAGAGAAACGCTGTTCCAATCACTCCTACTTTGAACAG
AGAACAATTGTCTACTTCTGAGGAAAACTCTAAGAAAACTGTTGATATGGAATCTACTGA
GGTTTTTACTAAGAAAACTAAGTTGACTGAGGAAGAGAAAAACAGATTGAACTTTTTGAA
GAAGATTTCTCAAAGATACCAAAAGTTCGCTTTGCCACAATACTTGAAGACTGTTTACCA
ACATCAAAAGGCTATGAAACCATGGATTCAACCTAAGACTAAAGTTATTCCTTACGTTAG
ATATTTGCATCCACACCCTCATTTGTCTTTCATGGCTATTCCACCTAAGAAAAACCAAGA
TAAGACTGAAATCCCAACTATTAATACTATTGCTTCTGGAGAGCCAACTTCTACTCCTAC
TATTGAAGCTGTTGAGTCTACTGTTGCTACTTTGGAAGCTTCTCCTGAAGTTATTGAGTC
TCCACCTGAGATTAATACTGTTCAAGTTACTTCTACTGCTGTTGGTTCTGGATCCCATCA
CCATCACCATCACTAA (SEQ ID NO:24).
DNA sequence encoding fusion protein comprising β-casein-linker-CMP:

ATGAGATTTCCATCTATTTTCACTGCTGTTTTGTTTGCTGCTTCTTCTGCTTTGGCTGCTC
CAGTTAACACTACTACTGAAGATGAGACTGCTCAAATTCCTGCTGAAGCTGTTATTGGTT
ACTCTGATTTGGAGGGAGATTTTGATGTTGCTGTTTTGCCTTTCTCTAACTCTACTAACA
ACGGTTTGTTGTTCATTAACACTACTATCGCTTCTATCGCTGCTAAGGAAGAGGGTGTTT
CTTTGGAAAAGAGAGAAGCTGAGGCTCACCATCACCACCATCACAGAGAATTGGAAGA
GTTGAACGTTCCAGGTGAAATTGTTGAGTCTTTGTCTTCTTCTGAAGAGTCTATCACTAG
AATTAATAAGAAAATTGAGAAGTTTCAATCTGAAGAGCAACAACAAACTGAAGATGAGTT
GCAAGATAAGATCCATCCATTCGCTCAAACTCAATCTTTGGTTTACCCATTCCCTGGTCC
AATTCCTAACTCTTTGCCTCAAAATATTCCACCTTTGACTCAAACTCCAGTTGTTGTTCCA
CCTTTCTTGCAACCTGAAGTTATGGGTGTTTCTAAGGTTAAAGAAGCTATGGCTCCAAA
GCATAAAGAGATGCCATTTCCTAAGTATCCAGTTGAACCTTTCACTGAGAGACAATCTTT
GACTTTGACTGATGTTGAAAACTTGCACTTGCCATTGCCTTTGTTGCAATCTTGGATGCA
TCAACCACACCAACCTTTGCCACCTACTGTTATGTTTCCACCTCAATCTGTTTTGTCTTT
GTCTCAATCTAAGGTTTTGCCAGTTCCTCAAAAAGCTGTTCCATACCCTCAAAGAGATAT
GCCAATTCAAGCTTTCTTGTTGTATCAAGAACCAGTTTTGGGTCCTGTTAGAGGTCCATT
TCCTATTATTGTTCATCCACACCCTCATTTGTCTTTCATGGCTATTCCACCTAAGAAAAAC
CAAGATAAGACTGAAATCCCAACTATTAATACTATTGCTTCTGGAGAGCCAACTTCTACT
CCTACTATTGAAGCTGTTGAGTCTACTGTTGCTACTTTGGAAGCTTCTCCTGAAGTTATT
GAGTCTCCACCTGAGATTAATACTGTTCAAGTTACTTCTACTGCTGTTGGTTCTGGATCC
CATCACCATCACCATCACTAA (SEQ ID NO:25).

In some embodiments, the sequence encoding the fusion protein is operably linked to a promoter. The skilled person will be aware of suitable promoters for expression of recombinant proteins in a given host. Preferred promoters may include those which are strong constitutive promoters in the presence of a particular carbon source (e.g. glucose, glycerol).

In some embodiments, the promoter is the GAP promoter (GAPp). The GAP promoter is the promoter from the *Pichia pastoris* TDH3 gene (Glyceraldehyde-3-phosphate dehydrogenase, GAPDH). In some embodiments the promoter is that of the *Pichia pastoris* GCW14 gene.

In some embodiments, the sequence further comprises a terminator. Preferred terminators may include those which are associated with high levels of expression. Suitable terminators include those of the AOX1 and TDH3 genes of *Pichia pastoris*, and the terminators of the PGK1, CYC1, RPL3, and BNA4 genes of *Saccharomyces cerevisiae*. In some embodiments, the terminator is that of the *Pichia pastoris* AOX1 gene.

The promoter, the sequence which encodes the recombinant fusion protein and the terminator (if present), which are comprised within the nucleic acid molecule, are together referred to as an "expression cassette". The invention thus also provides an expression cassette.

In some embodiments, the nucleic acid molecule further comprises one or more homology regions (e.g. two homology regions). These can be used to direct integration of the sequence encoding the recombinant fusion protein, or the expression cassette, into the genome of a host cell. The skilled person will be capable of designing suitable homology regions using their common general knowledge. For example, the homology regions may be designed based on integration loci that have been successfully used previously, loci that are known to result in higher expression, and/or loci where integration does not disturb endogenous expression.

In some embodiments, the nucleic acid molecule, or the sequence encoding the recombinant fusion protein, is codon-optimised for expression in a desired host cell, such as a bacterial cell or a eukaryotic cell (e.g. a yeast or a plant cell). In some embodiments the nucleic acid molecule, or the sequence encoding the recombinant fusion protein, is codon-optimised for expression in *Pichia pastoris.*

The invention further provides a vector comprising the nucleic acid molecule described herein.

The vector may be a plasmid, a cosmid, or a viral vector.

In some embodiments, the vector is an expression vector for expression of the recombinant fusion protein in a host cell. The expression vector may be a plasmid or a cosmid. It will be appreciated that an expression vector is a vector (e.g. a DNA vector) which can be introduced into a host cell and which will remain autonomous (i.e. it will replicate within the host cell and remain as an individual entity rather than be integrated in the genome) and from which proteins will be expressed.

In some embodiments, the vector is a cloning vector, such as a plasmid.

Also provided is a host cell comprising a nucleic acid molecule (e.g. an expression cassette) or a vector (e.g. an expression vector or a cloning vector) as described herein.

In some embodiments, the host cell comprises an expression cassette which is integrated into the genome of the host cell, wherein the expression cassette comprises a promoter, a sequence encoding the recombinant nucleic acid molecule operably linked to the promoter and, optionally, a terminator.

Integration of the expression cassette into the host cell genome can be carried out using standard techniques known to those skilled in the art, and as described herein. For example, the host cell may be co-transformed with: (i) a donor DNA molecule comprising the expression cassette located between two homology regions; and (ii) a plasmid comprising an RNA-guided endonuclease (CRISPR) system. The plasmid may further comprise an antibiotic resistance gene, enabling the selection of host cells which have been successfully transformed with the plasmid DNA. The proportion of plasmid and donor DNA in the transformation mix may be controlled so as to achieve a high probability that any host cell transformed with the plasmid is also transformed with the donor DNA molecule. The RNA-guided endonuclease (CRISPR) system ensures that the host cell genome has a double-strand cut at a target location, thereby promoting homology-driven recombination of the donor DNA molecule such that the expression cassette is integrated into the host cell genome.

In some embodiments, the host cell comprises multiple expression cassettes which are integrated into the host cell genome. In some embodiments, the host cell genome comprises two, three, four or more expression cassettes. All of the expression cassettes may be the same. For example, each expression cassette may comprise a sequence encoding the same recombinant fusion protein. Insertion of multiple copies of the same gene (i.e. the sequence encoding the recombinant fusion protein) can be useful to increase protein yield. In some embodiments, each expression cassette is different. For example, each expression cassette may comprise a sequence encoding a different recombinant fusion protein.

In embodiments wherein the host cell comprises multiple expression cassettes, each expression cassette may comprise a different promoter (and, optionally a different terminator). This may be advantageous to reduce the risk of unwanted recombinations. The relative abundance of each recombinant protein expressed may also be controlled through the use of different promoters.

Such host cells may be prepared by transforming the host cell with: (i) a first donor DNA molecule comprising a first expression cassette located between a first pair of homology regions; and (ii) a first plasmid. Once integration of the first expression cassette into the host cell genome is complete, the first plasmid can be lost by culturing the host cells without selective pressure (i.e. in the absence of the antibiotic which the first plasmid confers resistance to). The host cell can then be transformed with: (i) a second donor DNA molecule comprising a second expression cassette located between a second pair of homology regions; and (ii) a second plasmid. The first and second expression cassettes may be the same (e.g. for integration of a second copy of DNA encoding the same recombinant fusion protein) or they may be different (e.g. for integration of DNA encoding a different recombinant fusion protein). The first and second pairs of homology regions can be different, so that the first and second expression cassettes are integrated into different regions of the host cell genome. The first and second plasmids can encode different guide RNAs.

In some embodiments, the host cell comprises an expression vector. The expression vector may comprise an expression cassette comprising a promoter, a sequence encoding the recombinant nucleic acid molecule operably linked to the promoter and, optionally, a terminator. The expression vector may be a plasmid. In some embodiments, the expression vector comprises multiple expression cassette, e.g. two, three, four or more expression cassettes. The expression cassettes may all be the same, or they may be different. For example, each expression cassette may comprise a sequence encoding a different recombinant fusion protein.

The host cell may be a bacterial cell or a eukaryotic cell. A eukaryotic host can be a plant cell, an animal cell or a fungal cell. A fungal cell can be a yeast cell.

In some embodiments, the host cell is a eukaryotic cell selected from *Saccharomyces spp., Kluyveromyces spp., Pichia spp., Aspergillus spp., Tetrahymena spp., Yarrowla spp., Hansenula spp., Blastobotrys spp., Candida spp., Zygosaccharomyces spp., Debaryomyces spp., Fusarium spp., and Trichoderma spp.*

In some embodiments, the host cell is *Pichia spp*, such as *Pichia pastoris.*

In some embodiments, the host cell is a plant cell, such as soy.

The invention also provides the use of the recombinant fusion protein described herein in the production of recombinant casein.

A method of producing a recombinant protein comprises culturing a host cell as described herein under conditions suitable for expression of a recombinant fusion protein as described herein.

Suitable conditions for culturing and expressing recombinant proteins in a given host cell will be known to those skilled in the art. For example, the expression of the recombinant fusion protein in a eukaryotic host cell, such as *Pichia pastoris,* may be carried out under the conditions described herein.

In some embodiments, the host cell expresses a recombinant fusion protein which comprises a cleavage site between the casein and the peptide. In such embodiments, the method may further comprise treating the recombinant fusion protein with an enzyme or a chemical which is capable of cleaving the recombinant fusion protein at the cleavage site, so as to separate the casein from the peptide.

In some embodiments, the method further comprises separating the recombinant fusion protein from the host cell culture, prior to treating the recombinant fusion protein with the enzyme or chemical.

Alternatively, the method may comprise treating the recombinant fusion protein with the enzyme or chemical in the host cell culture (i.e. by adding the enzyme or chemical to the culture), and then separating the cleaved casein from the culture.

The enzyme may be any suitable enzyme, such as those described herein. In some embodiments, the enzyme is chymosin.

In some embodiments, the method may further comprise inactivating the cleaving enzyme. This may be achieved by thermally treating the enzyme e.g. by heating. For example, the method may comprise heating a suspension comprising the recombinant fusion proteins. The suspension may be the host cell culture, or it may comprise the recombinant fusion proteins after separating the proteins from the cell culture. containing the enzyme. Inactivating the cleaving enzyme may help to prevent or reduce degradation of the separated casein. In some embodiments in which the separated casein is isolated (e.g. from the cell culture) rapidly after cleavage is performed, thermal inactivation of the cleaving enzyme may not be required.

The method may further comprise isolating and/or purifying the separated casein.

Isolating the separated casein from the host cell culture may comprise one or more of the following techniques: centrifugation; precipitation (e.g. by adjusting the pH and/or salt composition of the culture medium); ultrafiltration; size exclusion chromatography; affinity chromatography; ion exchange chromatography; reverse phase chromatography; and immunoprecipitation.

The invention thus provides a recombinant casein obtained by the method described hereinabove. The recombinant casein may be recombinant α-S1 casein, recombinant α-S2 casein or recombinant β-casein.

It will be appreciated that the recombinant casein may comprise, in addition to the amino acid sequence of the casein protein itself, one or more additional features that were present in the recombinant fusion protein, such as one or more tags, a signal sequence fragment, and/or a linker or portion thereof.

In some embodiments, the recombinant casein comprises at least a portion of a linker. The linker, or portion thereof, may be located at the C-terminus or the N-terminus of the recombinant casein. In some embodiments the linker, or portion thereof, is located at the C-terminus of the recombinant casein. In some embodiments, the recombinant casein comprises at least a portion of a cleavage site, such as an enzyme cleavage site (e.g. a chymosin cleavage site).

In some embodiments, the recombinant casein comprises a fragment of a signal sequence. Such a fragment may be generated by cleavage of the signal sequence during or immediately after translocation of the recombinant fusion protein into the host cell's secretory pathway. The signal sequence fragment may be located at the N-terminus of the recombinant casein.

Thus, in some embodiments the recombinant casein may comprise the structure:
Signal sequence fragment - casein - linker (or portion thereof).

In some embodiments, the recombinant α-S1 casein comprises the sequence of SEQ ID NO: 26:

EAEAHHHHHHRPKHPIKHQGLPQEVLNENLLRFFVAPFPEVFGKEKVNELSKDIGSESTED
QAMEDIKQMEAESISSSEEIVPNSVEQKHIQKEDVPSERYLGYLEQLLRLKKYKVPQLEIVPN
SAEERLHSMKEGIHAQQKEPMIGVNQELAYFYPELFRQFYQLDAYPSGAWYYVPLGTQYT
DAPSFSDIPNPIGSENSEKTTMPLWHPHPHLSF (SEQ ID NO:26).

In some embodiments, the recombinant α-S2 casein comprises the sequence of SEQ ID NO: 27:

EAEAHHHHHHKNTMEHVSSSEESIISQETYKQEKNMAINPSKENLCSTFCKEVVRNANEEE
YSIGSSSEESAEVATEEVKITVDDKHYQKALNEINQFYQKFPQYLQYLYQGPIVLNPWDQVK
RNAVPITPTLNREQLSTSEENSKKTVDMESTEVFTKKTKLTEEEKNRLNFLKKISQRYQKFAL
PQYLKTVYQHQKAMKPWIQPKTKVIPYVRYLHPHPHLSF (SEQ ID NO:27).

In some embodiments, the recombinant β-casein comprises the sequence of SEQ ID NO: 28:

EAEAHHHHHHRELEELNVPGEIVESLSSSEESITRINKKIEKFQSEEQQQTEDELQDKIHPFA
QTQSLVYPFPGPIPNSLPQNIPPLTQTPVVVPPFLQPEVMGVSKVKEAMAPKHKEMPFPKY
PVEPFTERQSLTLTDVENLHLPLPLLQSWMHQPHQPLPPTVMFPPQSVLSLSQSKVLPVPQ
KAVPYPQRDMPIQAFLLYQEPVLGPVRGPFPIIVHPHPHLSF (SEQ ID NO:28).

In the sequences of SEQ ID NOs 17-28 shown above, the underlined portions indicate tags or, in the case of the nucleic acid sequences, a sequence encoding a tag. It will be appreciated that one or both of the tags may be omitted from the fusion protein. Accordingly, the present invention also provides corresponding sequences without one or both of the tags (i.e. the same sequence but lacking one or both of the underlined portions).

The invention further provides the use of the recombinant fusion protein described herein in the production of a food composition.

A method of making a food composition comprises:
(i) providing a recombinant fusion protein as described herein;
(ii) separating the casein from the peptide; and
(iii) using the separated casein to make a food composition.

In some embodiments, the recombinant fusion protein comprises a cleavage site between the casein and the peptide. In such embodiments, the step of separating the casein from the peptide comprises treating the recombinant fusion protein with an enzyme or a chemical capable of cleaving the recombinant fusion protein so as to separate the casein from the peptide.

In some embodiments, the food composition is an alternative food composition, such as an alternative dairy food composition.

As used herein, the term "alternative" refers to a composition that comprises at least one isolated, recombinant casein protein. The recombinant casein may be present in the composition instead of, or in addition to, its equivalent animal-derived protein. For example, an alternative dairy composition may comprise a recombinant α-S1 casein according to the invention instead of an animal-derived α-S1 casein. The "animal derived" will be understood as referring to a natural casein protein that comes from an animal, e.g. in animal milk.

In some embodiments, the alternative food composition does not comprise any animal-derived caseins.

In some embodiments, the alternative food composition comprises one, two or three recombinant casein proteins in accordance with the invention. For example, the alternative food composition may comprise recombinant α-S1 casein and recombinant α-S2 casein. The alternative composition may comprise recombinant α-S1 casein and recombinant β-casein. The alternative food composition may comprise recombinant α-S2 casein and recombinant β-casein. In some embodiments, the alternative food composition comprises recombinant α-S1 casein, recombinant α-S2 casein and recombinant β-casein.

The alternative food composition may further comprise recombinant κ-casein.

The alternative food composition may comprise one or more additional ingredients selected from: beta-lactoglobulin, alpha-lactalbumin, serum albumin, lactoferrin, immunoglobulin, vegetal protein, salt (e.g. sodium chloride or potassium chloride), starch, sugar (e.g. lactose, sucrose, glucose, fructose and/or galactose), water, flavouring agent, preservative, emulsifier (e.g. lecithin), fats.

In some embodiments, the alternative dairy composition is a milk composition, a butter composition, a yoghurt composition, a sour cream composition, a creme fraiche composition, a baby formula composition, a cream composition, an ice-cream composition, a custard composition, or a cheese composition.

The invention thus provides a food composition obtained by the method described herein above.

It will be appreciated that any aspect or embodiment described herein may be combined with any other aspect or embodiment as appropriate, unless otherwise stated.

### Example 1

### Preparation constructs for expression of recombinant fusion proteins

1. Protein and DNA sequences of bovine caseins were obtained from databases. The codon usage of protein coding DNA was optimized for expression in *Pichia pastoris.*
2. DNA sequences coding for a promoter (GAPp: promoter of the *Pichia pastoris* TDH3 gene), *S*. *cerevisiae* α-mating factor (MF-α) signal sequence (SS), caseins (αS1,αS2, β, K), tags and terminator (AOX1t: terminator of the Pichia pastoris AOX1 gene)were produced by *in vitro* DNA synthesis and onboarded in a cloning vector (a derivative of pUC19 which retains the AmpR-Linker-Ori sequence, and in which the LacZ and multiple cloning site (MCS) region is replaced by a MCS partly derived from pUCmu (Staal et al. 2019 Biotechniques 66(6); 254-259) modified to include at its centre Aarl and Bsgl sites repeated and separated by a Sall site.).
3. A ca. 1000 bp region upstream of the genomic loci targeted for insertion was amplified from genomic DNA by PCR using a standard PCR protocol, thereby creating a first homology region (HRL).
4. A ca. 1000 bp region downstream of the genomic loci targeted for insertion was amplified from genomic DNA by PCR using a standard PCR protocol, thereby creating a second homology region (HRR).
   The primers used in steps 3 and 4 to create the homology regions were designed with overhangs to allow assembly of the resulting fragments and a linearized cloning vector through a standard Gibson Assembly protocol (i.e. the reverse primer for HRL has an overhang that overlaps the forward primer for HRR, the forward primer for HRL has an overhang that overlaps the reverse primer from step 5 and the reverse primer for HRR has an overhang that overlaps the forward primer from step 5). Furthermore the forward primer for HRL and the reverse primer for HRR were designed to introduce type IIS restriction sites (e.g. Aarl, Bsgl) such that recognition occurs outside the homology region but cleavage occurs within.
5. A cloning vector (pUC19) was linearized by PCR with primers that amplify the entire vector except the MCS.
6. The DNA fragments resulting from steps 3, 4 and 5 where assembled to form a circular vector pUC19-HRL-HRR by a standard Gibson Assembly protocol.
7. A DNA fragment encoding the expression cassette Promoter-SS-Casein-Tag-Terminator was amplified by PCR for each of casein αS1, αS2, β and K, from the vectors of step 2, wherein "SS" refers to a signal sequence.
8. The vector of step 6 was linearized by PCR with primers containing overhangs designed to overlap the first bases of the promoter and last bases of the terminator from step 7 (i.e. a reverse primer binding the 3' end of HRL with an overhang complementary to the 5' end of the promoter and a forward primer binding the 5' end of HRR with an overhang complementary to the 3' end of the terminator).
9. The linearized vector from step 8 was assembled into a circular vector with each of the DNA fragments from step 7 by a standard Gibson Assembly protocol, resulting in the circular vectors:
   a) pUC19-HRL-Promoter-SS-CSN1S1-Tag-Terminator-HRR
   b) pUC19-HRL-Promoter-SS-CSN1S2-Tag-Terminator-HRR
   c) pUC19-HRL-Promoter-SS-CSN2-Tag-Terminator-HRR
   d) pUC19-HRL-Promoter-SS-CSN3-Tag-Terminator-HRR
   where CSN1S1 is the gene encoding αS1 casein, CSN1S2 is the gene encoding αS2 casein, CSN2 is the gene encoding β casein, CSN3 is the gene encoding K casein and where K casein comprises the regions: para-casein K - chymosin recognition sequence (CRS)-caseinomacropeptide (CMP).
10. Vector (d) from step 9 was linearized by PCR with a reverse primer binding on the 3' end of the SS and containing an overhang coding for a tag and a forward primer binding on the 5' end of the CRS resulting in a linear DNA molecule that excludes the para Kappa Casein fragment: CRS-CMP-Tag-Terminator-HRR-pUC19-HRL-Promoter-SS-Tag
11. DNA encoding caseins αS1, αS2, and β (without SS or Tag) was amplified by PCR from the plasmids of step 9. The primers used were designed to introduce an N-terminal tag and contain overhangs to allow Gibson Assembly with the linear DNA molecule of step 10 (i.e. a forward primer binding on the 5' end of each casein with an overhang complementary to the tag introduced by the reverse primer of step 10, and a reverse primer binding the 3' end of each casein with an overhang complementary to the 5' end of the CRS).
12. The linearized vector from step 10 was assembled into a circular vector with each of the DNA molecules from step 11 by a standard Gibson Assembly protocol resulting in the circular vectors:
   e) pUC19-HRL-Promoter-SS-Tag-CSN1S1-linker-CMP-Tag-Terminator-HRR
   f) pUC19-HRL-Promoter-SS-Tag-CSN1S2-linker-CMP-Tag-Terminator-HRR
   g) pUC19-HRL-Promoter-SS-Tag-CSN2-linker-CMP-Tag-Terminator-HRR where the linker comprises the chymosin recognition sequence (CRS).
13. Donor DNA was separated from backbone DNA (pUC19) by restriction of the vectors of steps 9 and 12 on the Aarl or Bsgl restriction sites introduced in steps 3 and 4 (on the 5' end of HRL and the 3' end of HRR) followed by agarose gel electrophoresis and extraction of the pertinent DNA band from the gel.

At all pertinent steps, correct amplification and assembly of DNA was verified by restriction analysis and/or sequencing.

Note that Gibson Assembly of only two fragments (backbone plus a single insert) can be inefficient compared to assembly of three or more fragments. To counter this, in steps 8 and 10 the backbone can be linearized into two partially overlapping fragments rather than a single full-length fragment.

### Transformation of host cells

14. Electro-competent *Pichia pastoris* cells (strain NRRL Y-11430 also known as CBS7435 or ATCC 76273) were prepared from exponentially growing cells by treatment with lithium acetate and sorbitol as described by Wu and Letchworth 2004 BioTechniques 36:152.

15. The electro-competent cells of step 14 were mixed with the donor DNA molecules of step 13 (individually) and with an episomal vector expressing an RNA guided endonuclease, guide RNA specific for the genomic loci targeted for insertion and an antibiotic resistance gene.

16. The mix of step 15 was transferred to an electroporation cuvette, subjected to a pulse (2 kV, 25 µF, 200 ohm), diluted with YPD growth media (Yeast extract 1%, Peptone 2%, Dextrose 2%), transferred to a fresh vial, allowed to recover for 2 hours at 30°C and spread on a YPD-Agar (2%) plate with an antibiotic for selection according to the resistance provided by the vector of step 15.

17. After 2-3 days of growth at 30°C single transformed colonies were picked from the selective YPD-Agar plate of step 16 and analysed by a standard colony PCR protocol with primers specific for the targeted genomic locus and the donor DNA.

18. Clones identified as positive (i.e. with a correctly integrated expression cassette) in step 17 were spread on non-selective YPD-Agar plates.

19. After 2 days at 30°C single colonies were picked from the plates of step 18 and spread on selective and non-selective plates. This step was repeated, passing cells from the non-selective plates onto selective and non-selective plates, until there was no growth on selective plates (indicating loss of the episomal vector).

### Host cell culture

20. Cells from step 19 were inoculated in 3 ml YPD and grown at 30 °C for ca. 8 h until mid-exponential phase.

21. The pre-cultures of step 20 were diluted to OD600 0.014 in 30 ml of BMDY media (2% peptone, 1% yeast extract, 100 mM potassium phosphate pH 6, 13.4 g/L YNB with ammonium sulphate, 2 % glucose 0.4 mg/L biotin) and grown for 25 h at 30 °C.

22. Samples from the cultures of step 21 where collected at various time points. Results are reported for samples at 23 hours of growth. For sampling, 4 ml was removed from the culture.

### Protein recovery

23. Secreted proteins in the samples of step 22 were separated from the cells by centrifugation, 5 min 1500 rcf.

### Determination of expression levels.

24. Recombinant casein or casein - CMP hybrids in a fraction of the supernatants from step 23 (e.g. 200 µl) were diluted as required (e.g. 500x) in PBS and quantified by a standard ELISA protocol using an HRP conjugated antibody specific against the tag (HIS), an HRP substrate (ABTS) and a calibration curve with a tagged protein of known concentration (HIS tagged FKBP12). To calculate protein concentration from detected absorbance, the differences in molecular weight of the standard and the various recombinant proteins as well as the number of tags on a single recombinant protein were taken into account.

### Results

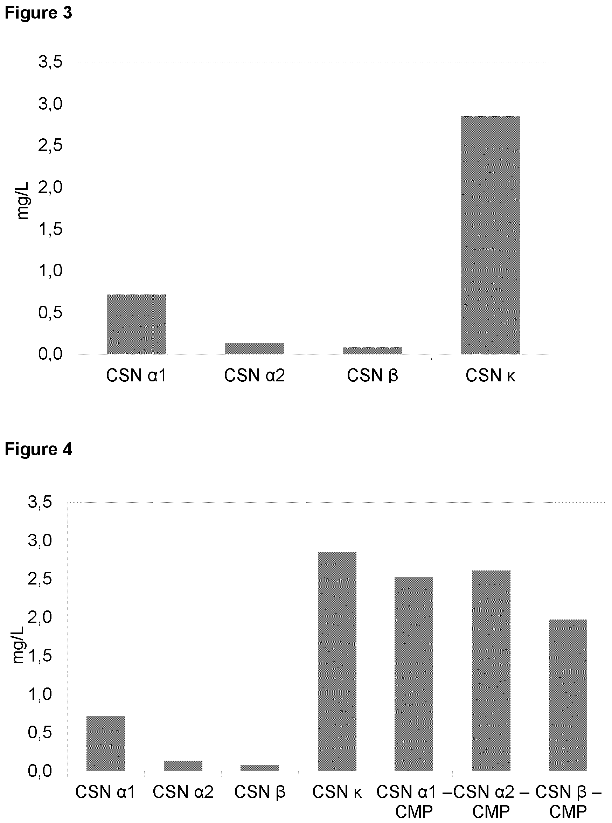

Caseins (CSN) alpha S1, alpha S2, beta and kappa were expressed, under the same conditions, from a strong promoter. As shown in Figure 3, the titre of casein kappa after 23 hours of culture was found to be four times that of casein alpha S1. The titres of caseins alpha S2 and beta were so low that they were almost at the detection limit (21 and 37 times less than that of casein kappa, respectively).

Constructs were prepared for expression of recombinant fusion proteins. With reference to Figure 2a, constructs were designed to include a DNA sequence encoding a recombinant fusion protein 10 comprising, from the C-terminus to the N-terminus: a MF-α signal sequence from *S*. *cerevisiae* 12; a first HIS tag 14; a casein protein 16; a linker 18 comprising a cleavage site; a caseinomacropeptide 20; and a second HIS tag 22. The DNA sequence was operably linked to promoter and terminator sequences. Homology regions to direct chromosomal integration are also fused to these DNA molecules. Separate constructs were prepared for the expression of each of α-S1 casein, α-2 casein and β-casein as fusion proteins.

The α-S1 casein - CMP fusion protein expressed in Example 1 is designated herein as SEQ ID NO: 17. The corresponding DNA sequence is designated as SEQ ID NO: 23.

The α-2 casein - CMP fusion protein expressed in Example 1 is designated herein as SEQ ID NO: 19. The corresponding DNA sequence is designated as SEQ ID NO: 24.

β-casein - CMP fusion protein expressed in Example 1 is designated herein as SEQ ID NO: 21. The corresponding DNA sequence is designated as SEQ ID NO: 25.

It will be appreciated that upon secretion of the fusion proteins from the host cell the signal sequence will be cleaved (indicated in Figure 2 by the dotted line), leaving behind a short fragment of the signal sequence 12' in the expressed protein. While the fusion proteins shown in the embodiment of Figure 2 comprised HIS tags, it will be appreciated that in other embodiments the recombinant fusion protein may contain different tags or no tags.

The resulting DNA constructs were separately transformed into *Pichia pastoris* host cells, which were cultured. This resulted in the host cells expressing the recombinant fusion proteins α-S1 casein-CMP, α-S2 casein-CMP or β-casein-CMP under the same conditions and from the same promoter as the (non-fusion) recombinant caseins from Figure 3. After 23 hours the titre of casein kappa was only 1.1 times that of α-S1 casein-CMP or α-S2 casein-CMP, and 1.4 times that of β-casein-CMP (Figure 4).

### Example 2 - cleavage of recombinant fusion protein

With reference to Figure 2, the recombinant fusion proteins may be cleaved at a cleavage site (CS) (e.g. using chymosin) so as to separate the caseinomacropeptide from the casein protein. In the embodiment shown in Figure 2b, the resulting cleaved casein protein 24 retains at least a portion of the linker 18'. Since cleavage of the CMP is carried out after secretion, the cleaved casein protein 24 comprises only a fragment 12' of the signal sequence 12.

Cleavage may be carried out according to the following protocol:
1. To supernatants containing a recombinant casein - CMP fusion, prepared as described in Example 1, a solution of cleavage enzyme (e.g.chymosin) in water is added to a final concentration of ca. 1 CU (coagulating unit) per gram of recombinant protein (as an example a standard commercial chymosin is provided as a solid with 1700 CU/g).
2. The mixture of step 1 is incubated for 20 min at 40 °C
3. For evaluation of cleavage 1 ml of the reaction from step 2 is stopped by addition of 250 µl trichloroacetic acid (TCA) 100%.
4. For recovery of recombinant protein the remainder of the reaction from step 2 is stopped by thermal treatment (2 min 60 °C).
5. Proteins and peptides are precipitated from the mix of step 3 by centrifugation at 4 °C, 10000 rcf for 15 minutes.
6. The supernatant from step 5 is completely removed.
7. The pellet from step 6 is washed once with ice cold acetone. Acetone is completely removed after centrifugation as in steps 5 and 6 and the resulting pellet is dried.
8. The dry pellet from step 7 is resuspended in Laemmli sample buffer with 0.1M dithiothreitol (DTT).
9. The mix of hybrids / proteins / peptides in the suspension from step 8 are separated through a standard SDS-PAGE electrophoresis protocol.
10. The separated hybrids / proteins / peptides from step 9 are analysed through a standard western blot protocol with an antibody specific for the tags (HIS) to evaluate the ratios of un-cleaved / cleaved / degraded proteins.
11. Proteins in the mix from step 4 are recovered in purified form from the growth/reaction media through: salt and pH-induced precipitation and/or ultrafiltration and/or a combination of chromatographic methods.

The invention may be described by any of the following clauses:
1. A recombinant fusion protein comprising:
   (a) a casein selected from α-S1 casein, α-S2 casein and β-casein; and
   (b) a peptide comprising a caseinomacropeptide (CMP), or a fragment thereof.
2. The recombinant fusion protein of clause 1, wherein the peptide decreases the hydrophobicity of the recombinant fusion protein relative to the hydrophobicity of the casein alone.
3. The recombinant fusion protein of clause 1 or clause 2, wherein the CMP or fragment thereof comprises an amino acid sequence of at least 20 residues.
4. The recombinant fusion protein of any preceding clause, wherein the CMP comprises an N-terminal and/or a C-terminal truncation.
5. The recombinant fusion protein of any preceding clause, wherein the CMP or fragment thereof comprises:
   (i) the amino acid sequence:
      X₃SGX₂PX₁ X₁X₁PTX₃X₂X₃X₃X₂S X₁X₃X₃X₁X₃X₂X₃X₁PX₂ X₃ (SEQ ID NO:5)
      wherein
      each X₁ is independently selected from S and T,
      each X₂ is independently selected from D and E, and
      each X₃ is independently selected from A, I, L and V, or
   (ii) an amino acid sequence which is at least 80% identical to SEQ ID NO: 5, optionally wherein at least 3 of the X₁ residues are retained.
6. The recombinant fusion protein of any preceding clause, wherein the CMP or fragment thereof comprises
   (i) an amino acid sequence selected from:
      X₃SGX₂PX₁ X₁X₁PTX₃X₂X₃X₃X₂S X₁X₃X₃X₁X₃X₂X₃X₁PX₂ X₃X₃X₂SPPX₂X₃NT X₃QX₃X₁STX₃X₃ (SEQ ID NO:6); and
      KNQX₂KTX₂X₃PX₁ X₃NTX₃X₃SGX₂PX₁ X₁X₁PTX₃X₂X₃X₃X₂S X₁X₃X₃X₁X₃X₂X₃X₁PX₂ X₃ (SEQ ID NO:7), or
   (ii) an amino acid sequence which is at least 80% identical to SEQ ID NO:6, optionally wherein at least 3 of the X₁ residues are retained, or
   (iii) an amino acid sequence which is at least 80% identical to SEQ ID NO:7, optionally wherein at least 3 of the X₁ residues are retained.
7. The recombinant fusion protein of any preceding clause, wherein the CMP or fragment thereof comprises
   (i) the amino acid sequence 
KNQX2KTX2X3PX1 X3NTX3X3SGX2PX1 X1X1PTX3X2X3X3X2S X1X3X3X1X3X2X3X1PX2
X3X3X2SPPX2X3NT X3QX3X1STX3X3 (SEQ ID NO:8); or
   (ii) an amino acid sequence which is at least 80% identical to SEQ ID NO:8, optionally wherein at least 3 of the X₁ residues are retained.
8. The recombinant fusion protein of part (ii) or (iii) of any one of clauses 5 to 8, wherein all of the X₁ residues are retained.
9. The recombinant fusion protein of any preceding clause, wherein the CMP or fragment thereof comprises
   (i) the amino acid sequence
      ASGEPT STPTIEAVES TVATLEASPE V (SEQ ID NO:9), or
   (ii) an amino acid sequence which is at least 80% identical to SEQ ID NO: 9..
10. The recombinant fusion protein of any preceding clause, wherein the CMP or fragment thereof comprises:
   (i) an amino acid sequence selected from: 
ASGEPT STPTIEAVES TVATLEASPE VIESPPEINT VQVTSTAV (SEQ ID NO:10);
KNQDKTEIPT INTIASGEPT STPTIEAVES TVATLEASPE V (SEQ ID NO:11); and
KNQDKTEIPT INTIASGEPT STPTIEAVES TVATLEASPE VIESPPEINT VQVTSTAV (SEQ
ID NO:12), or
   (ii) an amino acid sequence which is at least 80% identical to SEQ ID NO:10, SEQ ID NO:11 or SEQ ID NO:12.
11. The recombinant fusion protein of any preceding clause wherein the CMP or fragment thereof comprises at least 3 glycosylation sites.
12. The recombinant fusion protein of any preceding clause wherein the peptide is capable of increasing the yield of the recombinant fusion protein relative to the yield of the equivalent casein alone when expressed under identical conditions.
13. The recombinant fusion protein of any preceding clause wherein the casein is α-S1 casein, optionally wherein the α-S1 casein comprises an amino acid sequence which is at least 70% identical to SEQ ID NO. 1.
14. The recombinant fusion protein of any one of clauses 1 to 12, wherein the casein is α-S2 casein, optionally wherein the α-S2 casein comprises an amino acid sequence which is at least 70% identical to SEQ ID NO. 2.
15. The recombinant fusion protein of any one of clauses 1 to 12, wherein the casein is β-casein, optionally wherein the β-casein comprises an amino acid sequence which is at least 70% identical to SEQ ID NO 3.
16. The recombinant fusion protein of any preceding clause, which further comprises a linker between the casein and the peptide.
17. The recombinant fusion protein of clause 16, wherein the linker comprises a cleavage site, optionally an enzyme cleavage site.
18. The recombinant fusion protein of clause 17, wherein the enzyme cleavage site is a protease cleavage site, optionally a chymosin cleavage site.
19. A nucleic acid molecule comprising a sequence encoding the recombinant fusion protein of any one of claims 1 to 18.
20. A vector comprising the nucleic acid molecule of clause 19.
21. A host cell comprising the nucleic acid of clause 19 or the vector of clause 20.
22. The host cell of clause 21, wherein the host cell is a plant cell, a bacterial cell, a fungal cell, a yeast cell or a mammalian cell, optionally wherein the yeast cell is *Pichia pastoris.*
23. The use of a recombinant fusion protein according to any one of clauses 1 to 18 in the production of a recombinant casein.
24. The use of a recombinant fusion protein according to any one of clauses 1 to 18 in the production of a food composition.
25. A method of producing a recombinant casein, the method comprising culturing the host cell of claim 21 under conditions suitable for expression of the recombinant fusion protein of any one of claims 1 to 18.
26. The method of clause 25, wherein the recombinant fusion protein comprises a linker comprising a cleavage site, and wherein the method further comprises treating the recombinant fusion protein with an enzyme or a chemical which is capable of cleaving the recombinant fusion protein at the cleavage site, so as to separate the casein from the peptide.
27. A recombinant casein obtained by the method of clause 26, optionally wherein the recombinant casein comprises at least a portion of a cleavage site.
28. A method of making a food composition, the method comprising:
   (i) providing a recombinant fusion protein according to any one of clauses 1 to 18;
   (ii) separating the casein from the peptide; and
   (iii) using the separated casein to make a food composition.
29. A food composition obtained by the method of any one of clauses 25 to 28.

## Claims

1. A recombinant fusion protein comprising:
(a) a casein selected from α-S1 casein, α-S2 casein and β-casein; and
(b) a peptide comprising a caseinomacropeptide (CMP), or a fragment thereof.

2. The recombinant fusion protein of claim 1, wherein the peptide decreases the hydrophobicity of the recombinant fusion protein relative to the hydrophobicity of the casein alone.

3. The recombinant fusion protein of claim 1 or claim 2, wherein the CMP or fragment thereof comprises an amino acid sequence of at least 20 residues.

4. The recombinant fusion protein of any preceding claim, wherein the CMP comprises an N-terminal and/or a C-terminal truncation.

5. The recombinant fusion protein of any preceding claim, wherein the CMP or fragment thereof comprises an amino acid sequence selected from Group A, Group B and Group C:
**Group A**
(i) the amino acid sequence:
X₃SGX₂PX₁ X₁X₁PTX₃X₂X₃X₃X₂S X₁X₃X₃X₁X₃X₂X₃X₁PX₂ X₃ (SEQ ID NO:5) or
(ii) an amino acid sequence which is at least 80% identical to SEQ ID NO: 5, optionally wherein at least 3 of the X₁ residues are retained;
or
**Group B**
(i) an amino acid sequence selected from:
X₃SGX₂PX₁ X₁X₁PTX₃X₂X₃X₃X₂S X₁X₃X₃X₁X₃X₂X₃X₁PX₂ X₃X₃X₂SPPX₂X₃NT X₃QX₃X₁STX₃X₃ (SEQ ID NO:6); and
KNQX₂KTX₂X₃PX₁ X₃NTX₃X₃SGX₂PX₁ X₁X₁PTX₃X₂X₃X₃X₂S X₁X₃X₃X₁X₃X₂X₃X₁PX₂ X₃ (SEQ ID NO:7), or
(ii) an amino acid sequence which is at least 80% identical to SEQ ID NO:6, optionally wherein at least 3 of the X₁ residues are retained, or
(iii) an amino acid sequence which is at least 80% identical to SEQ ID NO:7, optionally wherein at least 3 of the X₁ residues are retained;
or
**Group C**
(i) the amino acid sequence 
KNQX2KTX2X3PX1 X3NTX3X3SGX2PX1 X1X1PTX3X2X3X3X2S X1X3X3X1X3X2X3X1PX2
X3X3X2SPPX2X3NT X3QX3X1STX3X3 (SEQ ID NO:8); or
(ii) an amino acid sequence which is at least 80% identical to SEQ ID NO:8, optionally wherein at least 3 of the X₁ residues are retained;
wherein
each X₁ is independently selected from S and T,
each X₂ is independently selected from D and E, and
each X₃ is independently selected from A, I, L and V.

6. The recombinant fusion protein of any preceding claim, wherein the CMP or fragment thereof comprises an amino acid sequence selected from Group E or Group F:
**Group E**
(i) the amino acid sequence
ASGEPT STPTIEAVES TVATLEASPE V (SEQ ID NO:9), or
(ii) an amino acid sequence which is at least 80% identical to SEQ ID NO: 9;
or
**Group F**
(i) an amino acid sequence selected from: 
ASGEPT STPTIEAVES TVATLEASPE VIESPPEINT VQVTSTAV (SEQ ID NO:10);
KNQDKTEIPT INTIASGEPT STPTIEAVES TVATLEASPE V (SEQ ID NO:11); and
KNQDKTEIPT INTIASGEPT STPTIEAVES TVATLEASPE VIESPPEINT VQVTSTAV (SEQ
ID NO:12), or
(ii) an amino acid sequence which is at least 80% identical to SEQ ID NO:10, SEQ ID NO:11 or SEQ ID NO:12.

7. The recombinant fusion protein of any preceding claim wherein the CMP or fragment thereof comprises at least 3 glycosylation sites.

8. The recombinant fusion protein of any preceding claim, which further comprises a linker between the casein and the peptide, wherein the linker comprises a cleavage site, optionally an enzyme cleavage site.

9. A nucleic acid molecule comprising a sequence encoding the recombinant fusion protein of any one of claims 1 to 8.

10. A vector comprising the nucleic acid molecule of claim 9.

11. A host cell comprising the nucleic acid of claim 9 or the vector of claim 10, wherein the host cell is a plant cell, a bacterial cell, a fungal cell, a yeast cell or a mammalian cell, optionally wherein the host cell is *Pichia pastoris.*

12. A method of producing a recombinant casein, the method comprising culturing the host cell of claim 11 under conditions suitable for expression of the recombinant fusion protein of any one of claims 1 to 8.

13. A recombinant casein obtained by the method of claim 12, optionally wherein the recombinant casein comprises at least a portion of a cleavage site.

14. A method of making a food composition, the method comprising:
(i) providing a recombinant fusion protein according to any one of claims 1 to 8;
(ii) separating the casein from the peptide; and
(iii) using the separated casein to make a food composition.

15. A food composition obtained by the method of claim 14.
